(19) Europäisches Patentamt
European Patent Office
Office européen des brevets

(11) Veröffentlichungsnummer: **0 030 377**
**B1**

# (12) EUROPÄISCHE PATENTSCHRIFT

(45) Veröffentlichungstag der Patentschrift: **22.02.84**

(51) Int. Cl.³: **C 07 C 177/00**, A 61 K 31/557

(21) Anmeldenummer: **80107657.1**

(22) Anmeldetag: **05.12.80**

(54) **9-Chlor-prostaglandinderivate, Verfahren zur Herstellung und Verwendung als Arzneimittel.**

(30) Priorität: **10.12.79 DE 2950027**

(43) Veröffentlichungstag der Anmeldung: **17.06.81 Patentblatt 81/24**

(45) Bekanntmachung des Hinweises auf die Patenterteilung: **22.02.84 Patentblatt 84/8**

(84) Benannte Vertragsstaaten: **AT BE CH DE FR GB IT LI LU NL SE**

(56) Entgegenhaltungen:
**EP - A - 0 000 207**
**US - A - 4 192 799**

**PROSTAGLANDINS, Band 16, Nr. 1, Juli 1978, Los Altos, Calif., US, C.E. ARRONIZ et al.: "Synthesis of ring halogenated prostaglandins", Seiten 47-65**
**CHEMISTRY, BIOCHEMISTRY AND PHARMACOLOGICAL ACTIVITY OF PROSTANOIDS, Pergamon Press, 1978, J.M. MUCHOWSKI: "Synthesis and bronchial dilator activity of some novel prostaglandin analogues (1)" Seiten 39-60**
**Binder et al., Prostaglandins, 6 (1974), 87 ff.**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Skuballa, Werner, Dr., Olwenstrasse 25, D-1000 Berlin 28 (DE)**
Erfinder: **Radüchel, Bernd, Dr., Gollanczstrasse 132, D-1000 Berlin 28 (DE)**
Erfinder: **Vorbrüggen, Helmut, Prof. Dr., Wilkestrasse 7, D-1000 Berlin 27 (DE)**
Erfinder: **Elger, Walter, Dr., Schorlemer Allee 12b, D-1000 Berlin 33 (DE)**
Erfinder: **Loge, Olaf, Dr., Bekassinen Weg 37, D-1000 Berlin 27 (DE)**
Erfinder: **Schillinger, Ekkehard, Dr., Im Amseltal 50, D-1000 Berlin 28 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 030 377 B1

## 9-Chlor-prostaglandinderivate, Verfahren zur Herstellung und Verwendung als Arzneimittel

Die Erfindung betrifft neue 9-Chlor-prostaglandinderivate, Verfahren zu ihrer Herstellung sowie ihre Verwendung als Arzneimittel.

Aus dem sehr umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metabolisch abgebaut werden. Alle Strukturveränderungen haben das Ziel, die Wirkungsdauer sowie die Selektivität der Wirksamkeit zu steigern.

Bekannt ist bereits 9-Desoxy-9-chlor-$PGF_{2\alpha}$, das von C. E. Arroniz et al. (Prosta glandius, 16 [1978], 47) und J. M. Muchowski in Chemistry, Biochemistry and Pharmcological Activity of Prostanoids (Pergamon Press, 1978) beschrieben wird. Außerdem werden in EU-A-0 000 207 Prostaglandine vom F-Typ beschrieben, die in 16-Stellung Methylgruppen oder Fluoratome tragen. Im Handel befindet sich Clorprosteno ein 17,18,19,20-Tetranor-16-m-chlorphenoxyprostaglandin $F_{2\alpha}$.

Es wurde nun gefunden, daß die neuen 9-Chlor-prostaglandinderivate eine hervorragende Wirkungsspezifität, bessere Wirksamkeit, längere Wirkungsdauer als natürliche Prostaglandine besitzen und besonders für die orale Applikation geeignet sind.

Die Erfindung betrifft 9-Chlor-prostaglandinderivate der allgemeinen Formel I

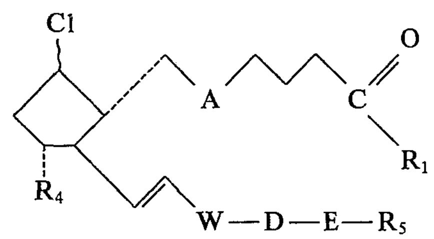

(I)

worin das 9-Chloratom $\alpha$- oder $\beta$-ständig sein kann,

- $R_1$ den Rest $OR_2$ mit $R_2$ in der Bedeutung eines Wasserstoffatoms, oder eines Alkyls mit 1–4 C-Atomen oder den Rest $NHR_3$ mit $R_3$ in der Bedeutung eines Wasserstoffatoms oder eines Carbon- oder Sulfonsäurerestes mit 1–10 C-Atomen,
- A eine $-CH_2-CH_2-$ oder cis-$CH=CH$-Gruppe,
- W eine freie oder funktionell abgewandelte Hydroxymethylengruppe, wenn die Gruppierung $-D-E-R_5$ nicht den Rest n-Pentyl darstellt, oder eine freie oder funktionell abgewandelte

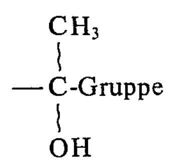

  wobei die OH-Gruppe $\alpha$- oder $\beta$-ständig sein kann,
- D und E gemeinsam eine direkte Bindung oder
- D eine geradkettige oder verzweigtkettige Alkylengruppe mit 1–5 C-Atomen, die gegebenenfalls durch Fluoratome substituiert ist, und
- E ein Sauerstoffatom oder eine direkte Bindung und
- $R_4$ eine freie oder funktionell abgewandelte Hydroxygruppe,
- $R_5$ eine gerad- oder verzweigtkettige, gesättigte oder ungesättigte Alkylengruppe mit 1–6 C-Atomen, die durch Halogen substituiert sein kann, oder eine gegebenenfalls durch 1–3 Halogenatome oder eine Trifluormethylgruppe substituierte Phenyl-Gruppe und

  falls $R_1$ die Bedeutung einer Hydroxylgruppe hat, deren Salze mit physiologisch verträglichen Basen bedeuten.

Als Alkylgruppen $R_2$ sind gerade oder verzweigte Alkylgruppen mit 1–4 C-Atomen zu betrachten, wie beispielsweise Methyl, Äthyl, Propyl, Butyl, Isobutyl, tert.-Butyl.

Als Säurerest $R_3$ kommen physiologisch verträgliche Säurereste in Frage. Bevorzugte Säuren sind organische Carbonsäuren und Sulfonsäuren mit 1–10 Kohlenstoffatomen, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seien Alkyl-, Hydroxy-, Alkoxy-, Oxo- oder

Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloressigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren kommen beispielsweise Methansulfonsäure, Äthansulfonsäure, Isopropylsulfonsäure, $\beta$-Chloräthansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Diäthylaminosulfonsäure, N,N-Bis($\beta$-Chloräthyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Die Hydroxygruppen in W und $R_4$ können funktionell abgewandelt sein, beispielsweise durch Verätherung oder Veresterung, wobei die freie oder abgewandelte Hydroxygruppe in W $\alpha$- oder $\beta$-ständig sein kann.

Als Äther- und Acylreste kommen die dem Fachmann bekannten Reste in Betracht. Bevorzugt sind leicht abspaltbare Ätherreste, wie beispielsweise der Tetrahydropyranyl-, Tetrahydrofuranyl-, $\alpha$-Äthoxyäthyl-, Trimethylsilyl-, Dimethyl-, tert.-Butyl-silyl- und Tribenzyl-silylrest. Als Acylreste kommen die gleichen wie für $R_3$ genannt in Frage, namentlich genannt seien beispielsweise Acetyl, Propionyl, Butyryl und Benzoyl.

Als Alkylgruppen $R_5$ kommen gerad- und verzweigtkettige, gesättigte und ungesättigte Alkylreste mit 1–6 C-Atomen in Frage, die gegebenenfalls durch Halogen substituiert sein können. Beispielsweise genannt seien Methyl-, Äthyl-, Propyl-, Butyl-, Isobutyl-, tert.-Butyl-, Pentyl-, Hexyl-, Butenyl-, Isobutenyl-, Propenyl-, Pentenylgruppen.

Die Phenylgruppe $R_5$ kann durch 1–3 Halogenatome oder eine Trifluormethylgruppe substituiert sein. Bevorzugt ist die Substitution in 3- und 4-Stellung am Phenylring zum Beispiel durch Fluor, Chlor oder Trifluormethyl.

Als Alkylengruppe D kommen geradkettige oder verzweigtkettige, gesättigte und ungesättigte Alkylenreste mit 1–5 C-Atomen in Frage, die gegebenenfalls durch Fluoratome substituiert sein können. Beispielsweise seien genannt: Methylen, Fluormethylen, Difluormethylen, Äthylen, 1.2-Propylen, Äthyläthylen, Trimethylen, Tetramethylen, Pentamethylen, 1.1-Difluoräthylen, 1-Fluoräthylen, 1-Methyltetramethylen, 1-Methyl-trimethylen, 1-Methylen-äthylen, 1-Methylen-tetramethylen.

Zur Salzbildung sind anorganische und organische Basen geeignet, wie sie dem Fachmann zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft ferner ein Verfahren zur Herstellung der erfindungsgemäßen 9-Chlor-prostaglandinderivate der allgemeinen Formel I, dadurch gekennzeichnet, daß man in an sich bekannter Weise eine Verbindung der allgemeinen Formel II

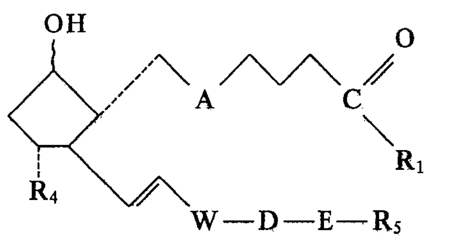

(II)

worin die OH-Gruppen $\alpha$- oder $\beta$-ständig sein können, $R_1$, A, D, E und $R_5$ die oben angegebene Bedeutung haben und freie OH-Gruppen in $R_4$ und W geschützt sind, a) über einen intermediären Sulfonsäureester mit einem Chlorid der allgemeinen Formel III $R_6Cl$, worin $R_6$ die Bedeutung Lithium, Natrium, Kalium oder Tetraalkylammonium mit Alkyl als gesättigtem $C_1-C_6$-Rest hat umsetzt oder b) mit dem Reagenz Tetrachlorkohlenstoff/Triphenylphosphin chloriert und gegebenenfalls anschließend in den nach a) oder b) erhaltenen Reaktionsprodukten in beliebiger Reihenfolge geschützte Hydroxygruppen freisetzt und/oder Doppelbindungen hydriert und/oder eine veresterte Carboxylgruppe ($R_1 = OR_2$) verseift und/oder eine freie Carboxylgruppe ($R_1 = OH$) verestert und/oder eine freie Carboxylgruppe ($R_1 = OH$) in ein Amid ($R_1 = NHR_3$) überführt.

Die Umsetzung der Verbindungen der allgemeinen Formel II zu einem 9-Sulfonsäureester erfolgt in

an sich bekannter Weise mit einem Alkylsulfonylchlorid oder Arylsulfonylchlorid in Gegenwart eines Amins wie beispielsweise Pyridin oder Triäthylamin bei Temperaturen zwischen −60° C und +100° C, vorzugsweise −20° C bis +50° C. Die nucleophile Substitution des 9-Sulfonats durch ein Chloratom erfolgt mit einem Alkalichlorid, vorzugsweise Lithiumchlorid oder Tetraalkylammoniumchlorid, vorzugsweise Tetrabutylammoniumchlorid in einem inerten Lösungsmittel wie beispielsweise Dimethylformamid, Dimethylacetamid, Dimethylsulfoxid, Dimethoxyäthan, Tetrahydrofuran usw. bei Temperaturen zwischen 0° C und 100° C, vorzugsweise 20° C bis 80° C.

Die Umsetzung der Verbindungen der allgemeinen Formel II zu den Verbindungen der allgemeinen Formel I mit Tetrachlorkohlenstoff und Triphenylphosphin erfolgt in einem inerten Lösungsmittel wie beispielsweise Dimethylformamid, Dimethylacetamid, Acetonitril, Methylenchlorid bei Temperaturen zwischen 0° C und 80° C, vorzugsweise 20° C bis 45° C.

Die Freisetzung der funktionell abgewandelten Hydroxygruppen erfolgt nach bekannten Methoden. Beispielsweise wird die Abspaltung von Hydroxyschutzgruppen, wie beispielsweise des Tetrahydropyranylrestes, in einer wäßrigen Lösung einer organischen Säure, wie z. B. Oxalsäure, Essigsäure, Propionsäure u. a., oder in einer wäßrigen Lösung einer anorganischen Säure, wie z. B. Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind z. B. Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20° C und 80° C durchgeführt.

Die Verseifung der Acylgruppen erfolgt beispielsweise mit Alkali- oder Erdalkali-carbonaten oder -hydroxyden in einem Alkohol oder in der wäßrigen Lösung eines Alkohols. Als Alkohole kommen aliphatische Alkohole in Betracht, wie z. B. Methanol, Äthanol, Butanol usw., vorzugsweise Methanol. Als Alkalicarbonate und -hydroxyde seien Kalium- und Natriumsalze genannt. Bevorzugt sind die Kaliumsalze.

Als Erdalkalicarbonate und -hydroxyde sind beispielsweise geeignet Calciumcarbonat, Calciumhydroxyd und Bariumcarbonat. Die Umsetzung erfolgt bei −10° C bis +70° C, vorzugsweise bei +25° C.

Die Einführung der Estergruppe $OR_2$ für $R_1$, bei welcher $R_2$ eine Alkylgruppe mit 1−10 C-Atomen darstellt, erfolgt nach den dem Fachmann bekannten Methoden. Die 1-Carboxy-verbindungen werden beispielsweise mit Diazokohlenwasserstoffen in an sich bekannter Weise umgesetzt. Die Veresterung mit Diazokohlenwasserstoffen erfolgt z. B. dadurch, daß man eine Lösung des Diazokohlenwasserstoffes in einem inerten Lösungsmittel, vorzugsweise in Diäthyläther, mit der 1-Carboxyverbindung in dem gleichen oder in einem anderen inerten Lösungsmittel, wie z. B. Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden (Org. Reactions, Bd. 8, Seiten 389−394 [1954]).

Sollen im Primärprodukt enthaltene C=C-Doppelbindungen reduziert werden, erfolgt die Hydrierung nach an sich bekannten Methoden.

Die Hydrierung der 5,6-Doppelbindung wird in an sich bekannter Weise bei tiefen Temperaturen, vorzugsweise bei etwa −20° C, in einer Wasserstoffatmosphäre in Gegenwart eines Erdmetallkatalysators durchgeführt. Als Katalysator ist zum Beispiel 10% Palladium auf Kohle geeignet.

Wird sowohl die 5,6- als auch die 13,14-Doppelbindung hydriert, so arbeitet man bei höherer Temperatur vorzugsweise bei etwa 20° C.

Die Prostaglandinderivate der allgemeinen Formel I mit $R_1$ in der Bedeutung einer Hydroxygruppe können mit geeigneten Mengen der entsprechenden anorganischen Basen unter Neutralisierung in ein Salz überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, z. B. Alkohol oder Aceton, das feste anorganische Salz.

Zur Herstellung eines Aminsalzes, die in üblicher Weise erfolgt, wird die PG-Säure z. B. in einem geeigneten Lösungsmittel, beispielsweise Äthanol, Aceton, Diäthyläther, Acetonitril oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die Einführung der Amidgruppe $NHR_3$ für $R_1$ erfolgt nach den dem Fachmann bekannten Methoden. Die Carbonsäuren der allgemeinen Formel I ($R_1$ = OH), werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$ = H) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen −30° C und +60° C, vorzugsweise bei 0° C bis 30° C.

Eine weitere Möglichkeit für die Einführung der Amidgruppe $NHR_3$ für $R_1$ besteht in der Umsetzung einer 1-Carbonsäure der allgemeinen Formel I ($R_1$ = OH), in der freie Hydroxygruppen gegebenenfalls intermediär geschützt sind, mit Verbindungen der allgemeinen Formel IV

$$O=C=N-R_3 \quad (IV)$$

worin $R_3$ die oben angegebene Bedeutung hat.

Die Umsetzung der Verbindung der allgemeinen Formel I ($R_1=OH$) mit einem Isocyanat der allgemeinen Formel IV erfolgt gegebenenfalls unter Zusatz eines tertiären Amins, wie z. B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen −80° C bis 100° C, vorzugsweise bei 0° C bis 30° C, vorgenommen werden.

Enthält das Ausgangsprodukt OH-Gruppen im Prostanrest, so werden diese OH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediär geschützt sind.

Im Vergleich zu PGE-Derivaten zecihnen sich die neuen 9-Chlor-Prostaglandine durch größere Stabilität aus.

Die neuen 9-Chlor-prostanderivate der allgemeinen Formel I sind wertvolle Pharmaka, da sie bei ähnlichem Wirkungsspektrum eine wesentlich verbesserte (höhere Spezifität) und vor allem wesentlich längere Wirkung aufweisen als die entsprechenden natürlichen Prostaglandine.

Die neuen Prostaglandin-Analoga wirken stark luteolytisch, d. h. zur Auslösung einer Luteolyse benötigt man wesentlich geringere Dosierungen als bei den entsprechenden natürlichen Prostaglandinen. Die neuen 9-Chlorprostaglandine erweisen sich am Meerschweinchen s.c. in der abortiven Wirkung $PGE_2$ überlegen.

Auch zur Auslösung von Aborten, insbesondere nach oraler Applikation, sind wesentlich geringere Mengen der neuen Prostaglandinanaloga im Vergleich zu den natürlichen Prostaglandinen erforderlich.

Bei der Registrierung der isotonischen Uteruskontraktion an der narkotisierten Ratte und am isolierten Rattenuterus zeigt sich, daß die erfindungsgemäßen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw. Ferner eignen sich die erfindungsgemäßen Prostaglandin-Derivate zur Cervixdilatation als Vorbereitung für diagnostische oder therapeutische Eingriffe.

Die gute Gewebespezifität der erfindungsgemäßen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine. Die erfindungsgemäßen Substanzen wirken auch bronchospasmolytisch. Außerdem bewirken sie eine Abschwellung der Nasenschleimhaut.

Die erfindungsgemäßen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandinsynthese − Inhibitoren) entgegen.

Einige der Verbindungen wirken blutdrucksenkend, regulierend bei Herzrhythmusstörungen und hemmend auf die Plättchenaggregation mit den sich daraus ergebenden Einsatzmöglichkeiten.

Für die medizinische Anwendung können die Wirkstoffe in eine für die Inhalation, für orale, parenterale oder lokale (z. B. vaginale) Applikation geeignete Form überführt werden.

Zur Inhalation werden zweckmäßigerweise Aerosollösungen hergestellt.

Für die orale Applikation sind beispielsweise Tabletten, Dragees oder Kapseln geeignet.

Für die parenterale Verabreichung werden sterile, injizierbare, wäßrige oder ölige Lösungen benutzt.

Für die vaginale Applikation sind z. B. Zäpfchen geeignet und üblich.

Die Erfindung betrifft damit auch Arzneimittel auf Basis der Verbindungen der allgemeinen Formel I und der üblichen Hilfs- und Trägerstoffe.

Die erfindungsgemäßen Wirkstoffe sollen in Verbindung mit den in der Galenik bekannten und üblichen Hilfsstoffen, z. B. zur Herstellung von Präparaten zur Auslösung eines Abortes, zur Zyklussteuerung, zur Einleitung einer Geburt oder zur Behandlung der Hypertonie dienen. Für diesen Zweck aber auch für die übrigen Anwendungen können die Präparate 0,01−50 mg der aktiven Verbindung enthalten.

Die folgenden Beispiele sollen die Erfindung näher erläutern, ohne daß damit eine Begrenzung vorgenommen wird.

## Beispiel 1

(5Z,13E)-(9R,11R,15R)-9-Chlor-11,15-dihydroxy-16-phenoxy-17,18,19,20-tetranor-5,13-prostadiensäure-methylester

Zur einer Lösung von 5,72 g (5Z,13E)-(9S,11R,15R)-9-hydroxy-11,15-bis-(tetrahydropyran-2-yloxy)-16-phenoxy-17,18,19,20-tetranor-5,13-prostadiensäuremethylester (hergestellt aus der entsprechenden Säure im Methylenchlorid mit 0,5molarer ätherischen Diazomethanlösung bei 0°C) in 17 ml Pyridin fügt man bei 0°C 3,8 g p-Toluolsulfonsäurechlorid, rührt 16 Stunden bei Eisbadtemperatur und 48 Stunden bei Raumtemperatur. Anschließend versetzt man mit 15 ml Wasser, rührt 2,5 Stunden bei Raumtemperatur, versetzt mit 1 l Äther, schüttelt nacheinander mit Wasser, 5%iger Schwefelsäure, 5%iger Natriumbicarbonatlösung und Wasser. Man trocknet über Magnesiumsulfat und dampft im Vakuum ein. Dabei erhält man 6,56 g des 9-Tosylats als farbloses Öl.

IR: 2950, 2875, 1733, 1600, 1590, 1365, 1240, 974/cm.

Eine Lösung aus 3,6 g des 9-Tosylats in 150 ml Dimethylformamid rührt man 4 Stunden mit 2,1 g Lithiumchlorid bei 60°C unter Argon. Anschließend gießt man auf 10%ige Natriumchloridlösung, extrahiert dreimal mit einer Mischung aus Äther/Hexan 1+1, schüttelt den organischen Extrakt dreimal mit Wasser, trocknet über Magnesiumsulfat und dampft im Vakuum ein.

Dabei erhält man 2,9 g der 9β-Chlorverbindung als farbloses Öl.

IR: 2955, 1734, 1603, 1591, 978/cm.

Zur Tetrahydropyranyläther-Abspaltung rührt man 2,9 g des vorstehend erhaltenen Rohproduktes 16 Stunden bei Raumtemperatur mit 80 ml einer Mischung aus Essigsäure/Wasser/Tetrahydrofuran (65+35+10) und dampft anschließend im Vakuum ein. Den Rückstand reinigt man durch Chromatographie an Kieselgel. Mit Äther als Elutionsmittel erhält man 1,1 g der Titelverbindung als farbloses Öl.

IR: 3600, 3400 (breit), 2940, 1730, 1603, 1591, 975/cm.

## Beispiel 2

(5Z,13E)-(9R,11R,15R)-9-Chlor-11,15-dihydroxy-16-phenoxy-17,18,19,20-tetranor-5,13,prostadiensäure

450 mg des nach Beispiel 1 hergestellten Methylesters rührt man 5 Stunden mit 15 ml einer Lösung aus Kaliumhydroxyd in Äthanol und Wasser (Herstellung: Man löst 2 g Kaliumhydroxid in 75 ml Äthanol und 25 ml Wasser). Anschließend säuert man mit 10%iger Zitronensäurelösung auf pH 4 an, extrahiert dreimal mit Methylenchlorid, wäscht den organischen Extrakt einmal mit Sole, trocknet über Magnesiumsulfat und dampft im Vakuum ein. Nach Chromatographie des Rückstandes an Kieselgel mit Methylenchlorid/Methanol als Elutionsmittel erhält man 405 mg der Titelverbindung als farbloses Öl.

IR: 3600, 3400 (breit), 2930, 2855, 1710, 1600, 1590, 971/cm.

## Beispiel 3

(13E)-(9R,11R,15R)-9-Chlor-11,15-dihydroxy-16-phenoxy-17,18,19,20-tetranor-13-prostensäure-methylester

Zu einer Lösung von 1,15 g (13E)-(9S,11R,15R)-9-hydroxy-11,15-bis-(tetrahydropyran-2-yloxy)-16-phenoxy-17,18,19,20-tetranor-13-prostensäuremethylester (hergestellt aus der entsprechenden Säure in Methylenchlorid mit 0,5molarer ätherischer Diazomethanlösung bei 0°C) in 3,5 ml Pyridin fügt man bei 0°C 720 mg p-Toluol-sulfonsäurechlorid, rührt 16 Stunden bei 0°C und 48 Stunden bei 25°C. Anschließend versetzt man mit 0,3 ml Wasser, rührt 2,5 Stunden bei 25°C, versetzt mit Äther, schüttelt nacheinander mit Wasser, 5%iger Schwefelsäure, 5%iger Natriumbicarbonatlösung und Wasser. Man trocknet über Magnesiumsulfat und dampft im Vakuum ein. Dabei erhält man 1,4 g des 9-Tosylats als farbloses Öl.

IR: 2950, 2873, 1732, 1600, 1591, 1495, 1365, 1240, 975/cm.

Eine Lösung aus 0,92 g des 9-Tosylats in 60 ml Dimethylformamid rührt man 4 Stunden mit 550 mg Lithiumchlorid bei 60°C unter Argon. Anschließend gießt man auf 10%ige Natriumchloridlösung, extrahiert dreimal mit einer Mischung aus Äther/Hexan 1+1, schüttelt den organischen Extrakt dreimal mit Wasser, trocknet über Magnesiumsulfat und dampft im Vakuum ein. Dabei erhält man 0,72 g der 9β-Chlorverbindung als farbloses Öl.

IR: 2955, 1733, 1602, 1590, 978/cm.

Zur Tetrahydropyranylätherabspaltung rührt man 0,72 g des vorstehend erhaltenen Rohproduktes 16 Stunden bei 25° mit 15 ml einer Mischung aus Essigsäure/Wasser/Tetrahydrofuran (65+35+10) und

dampft anschließend im Vakuum ein. Den Rückstand reinigt man durch Chromatographie an Kieselgel. Mit Äther als Elutionsmittel erhält man 0,29 g der Titelverbindung als farbloses Öl.

IR: 3600, 3400 (breit), 2945, 1731, 1602, 1590, 976/cm.

## Beispiel 4

(13E)-(9R,11R,15R)-9-Chlor-11,15-dihydroxy-16-phenoxy-17,18,19,20-tetranor-13-prostensäure

In Analogie zu Beispiel 2 erhält man aus 0,25 g des nach Beispiel 3 hergestellten Methylesters 0,19 g der Titelverbindung als farbloses Öl.

IR: 3600, 3420 (breit), 2935, 2837, 1710, 1600, 1592, 972/cm.

## Beispiel 5

(5Z,13E)-(9R,11R,15R)-9-Chlor-11,15-dihydroxy-16-(m-chlorphenoxy)-17,18,19,20-tetranor-5,13-prostadiensäure-methylester

Zu einer Lösung von 2,95 g (5Z,13E)-(9S,11R,15R)-9-hydroxy-11,15-bis-(tetrahydropyran-2-yloxy)-16-(m-chlorphenoxy)-17,18,19,20-tetranor-5,13-prostadiensäure-methylester (hergestellt aus der entsprechenden Säure in Methylenchlorid mit 0,5molarer Diazomethanlösung bei 0° C) in 8 ml Pyridin fügt man bei 0° C 1,9 g p-Toluolsulfonsäurechlorid, rührt 16 Stunden bei Eisbadtemperatur und 48 Stunden bei Raumtemperatur. Anschließend versetzt man mit 5 ml Wasser, rührt 2,5 Stunden bei Raumtemperatur, versetzt mit 0,4 l Äther, schüttelt nacheinander mit Wasser, 5%iger Schwefelsäure, 5%iger Natriumbicarbonatlösung und Wasser. Man trocknet über Magnesiumsulfat und dampft im Vakuum ein. Dabei erhält man 3,4 g des 9-Tosylats als farbloses Öl.

IR: 2955, 2873, 1733, 1600, 1588, 972/cm.

Eine Lösung aus 3,4 g des 9-Tosylats in 150 ml Dimethylformamid rührt man 4 Stunden mit 2,0 g Lithiumchlorid bei 60° C unter Argon. Anschließend gießt man auf 10%ige Natriumchloridlösung, extrahiert dreimal mit einer Mischung aus Äther/Hexan 1+1, schüttelt den organischen Extrakt dreimal mit Wasser, trocknet über Magnesiumsulfat und dampft im Vakuum ein.

Dabei erhält man als Rohprodukt 2,7 g der 9$\beta$-Chlorverbindung als farbloses Öl.

IR: 2955, 1733, 1600, 1587, 975/cm.

Zur Tetrahydropyranyläther-Abspaltung rührt man 2,7 g des vorstehend erhaltenen Rohproduktes 16 Stunden bei Raumtemperatur mit 70 ml einer Mischung aus Essigsäure/Wasser/Tetrahydrofuran (65+35+10) und dampft anschließend im Vakuum ein. Nach Reinigung des Rückstandes an Kieselgel erhält man mit Äther als Elutionsmittel 0,95 g der Titelverbindung als farbloses Öl.

IR: 3600, 3420 (breit), 2940, 1732, 1600, 1588, 976/cm.

## Beispiel 6

(5Z,13E)-(9R,11R,15R)-9-Chlor-11,15-dihydroxy-16-(m-chlorphenoxy)-17,18,19,20-tetranor-5,13-prostadiensäure

In Analogie zu Beispiel 2 erhält man aus 510 mg des nach Beispiel 5 hergestellten Methylesters 460 mg der Titelverbindung als farbloses Öl.

IR: 3600, 3400 (breit), 2940, 2860, 1710, 1600, 1588, 973/cm.

## Beispiel 7

(13E)-(9R,11R,15R)-9-Chlor-11,15-dihydroxy-16-(m-chlorphenoxy)-17,18,19,20-tetranor-13-prostensäure-methylester

Eine Lösung von 600 mg (13E)-(9S,11R,15R)-9-hydroxy-11,15-bis-(tetrahydropyran-2-yloxy)-16-(m-chlorphenoxy)-17,18,19,20-tetranor-13-prostensäuremethylester (hergestellt durch Hydrierung aus (5Z,13E)-(9S,11R,15R)-9-hydroxy-11,15-bis-(tetrahydropyran-2-yloxy)-16-(m-chlorphenoxy)-17,18,19,20-tetranor-5,13-prostandiensäuremethylester in Essigester bei 20° mit Palladium 10%ig auf Kohle und einem Äquivalent Wasserstoff) in 2 ml Pyridin versetzt man bei 0° C mit 390 mg p-Toluolsulfonsäurechlorid, rührt 16 Stunden bei Eisbadtemperatur und 48 Stunden bei Raumtemperatur. Anschließend versetzt man mit 0,5 ml Wasser, rührt 3 Stunden bei Raumtemperatur, verdünnt mit Äther, schüttelt nacheinander mit Wasser, 5%iger Schwefelsäure, 5%iger Natriumbicarbonatlösung und Wasser. Man trocknet über Magnesiumsulfat und dampft im Vakuum ein. Dabei erhält man 680 mg des 9-Tosylats

als farbloses Öl.

IR: 2955, 2873, 1732, 1600, 1588, 974/cm.

Eine Lösung aus 680 mg des 9-Tosylats in 15 ml Dimethylformamid rührt man 4 Stunden mit 220 mg Lithiumchlorid bei 60° C unter Argon. Anschließend gießt man auf 10%ige Natriumbicarbonatlösung, extrahiert dreimal mit einer Mischung aus Äther/Hexan 1+1, schüttelt den organischen Extrakt dreimal mit Wasser, trocknet über Magnesiumsulfat und dampft im Vakuum ein. Dabei erhält man als Rohprodukt 520 mg der 9β-Chlorverbindung als Öl.

IR: 2955, 1732, 1600, 1588, 975/cm.

Zur Tetrahydropyranyläther-Abspaltung rührt man 520 mg des vorstehend erhaltenen Rohproduktes 16 Stunden bei Raumtemperatur mit 10 ml einer Mischung aus Essigsäure/Wasser/Tetrahydrofuran (65+35+10) und dampft anschließend im Vakuum ein. Nach Reinigung des Rückstandes an Kieselgel erhält man mit Äther als Elutionsmittel 225 mg der Titelverbindung als farbloses Öl.

## Beispiel 8

### (13E)-(9R,11R,15R)-9-Chlor-11,15-dihydroxy-16-(m-chlorphenoxy)-17,18,19,20-tetranor-13-prostensäure

In Analogie zu Beispiel 2 erhält man aus 200 mg des nach Beispiel 7 hergestellten Methylesters 165 mg der Titelverbindung als farbloses Öl.

IR: 3600, 3420 (breit), 2944, 2860, 1710, 1600, 1588, 975/cm.

## Beispiel 9

### (5Z,13E)-(9R,11R,15R)-9-Chlor-11,15-dihydroxy-16,16-dimethyl-5,13-prostadiensäure-methylester

Eine Lösung aus 1,3 g (5Z,13E)-(9S,11R,15R)-16,16-Dimethyl-9-hydroxy-11,15-bis-(tetrahydropyran-2-yloxy-5,13-prostadiensäuremethylester, 800 mg Triphenylphosphin und 370 mg Tetrachlorkohlenstoff in 6 ml Acetonitril rührt man 2 Stunden bei 80° C. Anschließend verdünnt man mit 40 ml Wasser, extrahiert dreimal mit einer Mischung aus Äther/Hexan (1+1), wäscht den organischen Extrakt mit Wasser, trocknet über Magnesiumsulfat und dampft im Vakuum ein. Nach Chromatographie des Eindampfrückstandes an Kieselgel erhält man mit Hexan/Äther 4+1 0,55 g der 9β-Chlorverbindung als farbloses Öl.

IR: 2960, 1733, 976/cm.

Zur Tetrahydropyranylätherspaltung rührt man 0,5 g der vorstehend erhaltenen 9β-Chlorverbindung mit 5 ml einer Mischung aus Essigsäure/Wasser/Tetrahydrofuran (65+35+10) und dampft anschließend im Vakuum ein. Den Rückstand reinigt man durch Chromatographie an Kieselgel. Mit Äther als Elutionsmittel erhält man 0,35 g der Titelverbindung als farbloses Öl.

IR: 3600, 3400 (breit), 2945, 1732, 976/cm.

## Beispiel 10

### (5Z,13E)-(9R,11R,15R)-9-Chlor-11,15-dihydroxy-16,16-dimethyl-5,13-prostadiensäure

In Analogie zu Beispiel 2 erhält man aus 0,3 g des nach Beispiel 9 hergestellten Methylesters 0,24 g der Titelverbindung als farbloses Öl.

IR: 3600, 3420 (breit), 2944, 1709, 975/cm.

## Beispiel 11

### (5Z,13E)-(9R,11R,16RS)-9-Chlor-11,15α-dihydroxy-16-methyl-5,13-prostadiensäure-methylester

Zu einer Lösung von 1,1 g (5Z,13E)-(9S,11R,16RS)-9-Hydroxy-16-methyl-11,15α-bis-(tetrahydropyran-2-yloxy)-5,13-prostadiensäure-methylester (hergestellt aus der entsprechenden Säure in Methylenchlorid mit 0,5molarer Diazomethanlösung bei 0° C) in 3,5 ml Pyridin fügt man bei 0° C 760 mg p-Toluolsulfonsäurechlorid, rührt 16 Stunden bei 0° C und 48 Stunden bei 25° C. Nach Aufarbeitung gemäß Beispiel 1 erhält man 1,4 g des 9-Tosylats als farbloses Öl.

IR: 2955, 2870, 1732, 975/cm.

Eine Lösung aus 1,4 g des 9-Tosylats in 60 ml Dimethylformamid rührt man 4 Stunden mit 840 mg

Lithiumchlorid bei 60°C unter Argon. Nach üblicher Aufarbeitung erhält man 1,1 g der 9β-Chlorverbindung als Öl.

IR: 2960, 1732, 975/cm.

Zur Tetrahydropyranyläther-Abspaltung rührt man 1,1 g des vorstehend erhaltenen Rohproduktes 16 Stunden bei Raumtemperatur mit 35 ml einer Mischung aus Essigsäure/Wasser/Tetrahydrofuran (65+35+10) und dampft anschließend im Vakuum ein. Nach Chromatographie des Rückstandes an Kieselgel erhält man mit Äther als Elutionsmittel 0,6 g der Titelverbindung als Öl.

IR: 3600, 3420 (breit), 2960, 1733, 976/cm.

## Beispiel 12

### (5Z,13E)-(9R,11R,16RS)-9-Chlor-11,15α-dihydroxy-16-methyl-5,13-prostadiensäure

In Analogie zu Beispiel 2 erhält man aus 0,5 g des nach Beispiel 11 hergestellten Methylesters 0,39 g der Titelverbindung als farbloses Öl.

IR: 3600, 3400 (breit), 2945, 1710, 976/cm.

## Beispiel 13

### (5Z,13E)-(9R,11R,15S)-9-Chlor-11,15-dihydroxy-15-methyl-5,13-prostadiensäure-methylester

Zu einer Lösung von 1 g (5Z,13E)-(9S,11R,15S)-9-Hydroxy-15-methyl-11,15-bis-(tetrahydropyran-2-yloxy)-5,13-prostadiensäure-methylester (hergestellt aus der entsprechenden Säure in Methylenchlorid mit 0,5molarer Diazomethanlösung) in 3,5 ml Pyridin fügt man bei 0°C 691 mg p-Toluolsulfonsäurechlorid, rührt 16 Stunden bei 0°C, dann 48 Stunden bei 25°C. Nach Aufarbeitung entsprechend dem Beispiel 1 erhält man 1,25 g des 9-Tosylats als farbloses Öl.

IR: 2950, 2870, 1735, 1601, 1365, 1175, 978, 905/cm.

Eine Lösung aus 1,20 g des 9-Tosylats in 50 ml Dimethylformamid rührt man 4,5 Stunden mit 720 mg Lithiumchlorid bei 60°C unter Argon. Man arbeitet entsprechend Beispiel 1 auf und erhält 900 mg der 9β-Chlorverbindung als Öl.

IR: 2955, 2868, 1735, 978/cm.

Zur Abspaltung der Schutzgruppen werden 800 mg der so erhaltenen 9β-Chlorverbindung mit 20 ml einer Mischung aus Essigsäure/Wasser/Tetrahydrofuran (65+35+10) 20 Stunden bei 25°C gerührt. Nach Eindampfen im Vakuum und Chromatographie des Rückstandes an Kieselgel mit Methylenchlorid erhält man 400 mg der Titelverbindung als Öl.

IR: 3600, 3420 (breit), 2955, 2870, 1735, 976/cm.

## Beispiel 14

### (5Z,13E)-(9R,11R,15S)-9-Chlor-11,15-dihydroxy-15-methyl-5,13-prostadiensäure

In Analogie zu Beispiel 2 erhält man aus 300 mg des nach Beispiel 13 hergestellten Methylesters 230 mg der Titelverbindung als Öl.

IR: 3600, 3400, 2950, 1710, 978/cm.

## Beispiel 15

### (5Z,13E)-(9R,11R,15R,16RS)-9-Chlor-11,15-dihydroxy-16-fluor-5,13-prostadiensäure-methylester

Eine Mischung aus 1,2 g (5Z,13E)-(9S,11R,15R,16RS)-9-Hydroxy-16-fluor-11,15-bis-(tetrahydropyran-2-yloxy)-5,13-prostadiensäure-methylester (hergestellt aus der entsprechenden Säure in Methylenchlorid mit 0,5molarer Diazomethanlösung bei 0°C), 800 mg p-Toluolsulfonsäurechlorid und 4 ml Pyridin rührt man 16 Stunden bei 0°C, anschließend 48 Stunden bei 25°C. Aufarbeitung entsprechend Beispiel 1 liefert 1,45 g des 9-Tosylats als Öl.

IR: 2952, 2870, 1732, 1601, 1360, 1170, 978, 906/cm.

1,25 g des so erhaltenen Tosylats werden mit 725 mg Lithiumchlorid in 50 ml Dimethylformamid unter Rühren 4 Stunden auf 60°C erhitzt. Nach Aufarbeitung entsprechend Beispiel 1 erhält man 925 mg der 9β-Chlorverbindung als Öl.

IR: 2950, 1735, 976/cm.

Zur Abspaltung der Schutzgruppen werden 900 mg der so erhaltenen 9β-Chlorverbindung mit 25 ml

einer Mischung aus Eisessig/Wasser/Tetrahydrofuran (65/35/10) 20 Stunden bei 25°C gerührt. Nach Eindampfen im Vakuum und Chromatographie des Rückstandes an Kieselgel mit Methylenchlorid erhält man 450 mg der Titelverbindung als Öl.

IR: 3605, 3420, 2952, 2868, 1735, 978/cm.

Beispiel 16

(5Z,13E)-(9R,11R,15R,16RS)-9-Chlor-11,15-dihydroxy-16-fluor-5,13-prostadiensäure

In Analogie zu Beispiel 2 erhält man aus 400 mg des nach Beispiel 15 hergestellten Methylesters 310 mg der Titelverbindung als Öl.

IR: 3600, 3400, 2952, 2860, 1712, 978/cm.

Beispiel 17

(5Z,13E)-(9S,11R,15R)-9-Chlor-11,15-dihydroxy-16-phenoxy-17,18,19,20-tetranor-5,13-prostadiensäure-methylester

Eine Lösung von 910 mg (5Z,13E)-(9R,11R,15R)-9-Hydroxy-11,15-bis-(tetrahydropyran-2-yloxy)-16-phenoxy-17,18,19,20-tetranor-prostadiensäuremethylester, 560 mg Triphenylphosphin und 260 mg Tetrachlorkohlenstoff in 5 ml Acetonitril wird 2 Stunden auf 80°C erhitzt. Zur Aufarbeitung verdünnt man mit 100 ml Wasser, extrahiert dreimal mit je 50 ml n-Heptan, wäscht den organischen Extrakt mit Sole, trocknet über Magnesiumsulfat und dampft im Vakuum ein. Den Rückstand chromatographiert man an Kiesel mit Hexan/Äther (2+1) und erhält 510 mg der 9$\alpha$-Chlorverbindung als Öl.

IR: 2955, 2870, 1735, 1600, 1590, 1100, 980/cm.

Zur Abspaltung der Schutzgruppen behandelt man die Substanz mit der Essigsäuremischung entsprechend Beispiel 1 und erhält 300 mg der Titelverbindung als Öl.

IR: 3600, 3400, 2950, 1735, 1601, 1590, 976/cm.

Der als Ausgangsmaterial verwendete (5Z,13E)-(9R,11R,15R)-9-Hydroxy-11,15-bis-(tetrahydropyran-2-yloxy)-16-phenoxy-17,18,19,20-tetranor-prostadiensäuremethylester wird wie folgt hergestellt:

Eine Lösung von 3 g des nach Beispiel 1 erhaltenen 9-Tosylats in 100 ml Dimethylsulfoxid wird mit 6 g Kaliumnitrit 3 h auf 60°C erhitzt. Nach Abkühlen verdünnt man mit 800 ml Wasser, extrahiert dreimal mit je 100 ml Hexan/Äther (2 : 1), wäscht die vereinigten Extrakte zweimal mit je 20 ml Sole, trocknet über Magnesiumsulfat und dampft im Vakuum ein. Der Rückstand wird an Kieselgel mit Hexan und steigendem Essigestergradienten gereinigt. Man erhält 1,5 g (9R)-9$\beta$-Hydroxy-Verbindung als Öl.

Beispiel 18

(5Z,13E)-(9,S11R,15R)-9-Chlor-11,15-dihydroxy-16-phenoxy-17,18,19,20 tetranor-5,13-prostadiensäure

In Analogie zu Beispiel 2 erhält man aus 250 mg der nach Beispiel 17 hergestellten Substanz 190 mg der Titelverbindung als Öl.

IR: 3600, 3410, 2960, 2870, 1710, 1600, 1588, 978/cm.

Beispiel 19

(5Z,13E)-(9R,11R,15R)-9-Chlor-11,15-dihydroxy-16-phenoxy-17,18,19,20-tetranor-5,13-prostadiensäure-methylsulfonamid

Eine Lösung von 200 mg (5Z,13E)-(9R,11R,15R)-9-Chlor-11,15-dihydroxy-16-phenoxy-17,18,19,20-tetranor-5,13-prostadiensäure in 5 ml Dimethylformamid wird bei 0°C mit 80 mg Chlorameisensäurebutylester und 60 mg Triäthylamin versetzt. Nach 30 Minuten werden 234 mg des Natriumsalzes von Methylsulfonamid (hergestellt aus Methylsulfonamid und Natriummethylat) und 2 ml Hexamethylphosphorsäuretriamid zugefügt und 3 Stunden bei 20°C gerührt. Anschließend gibt man das Reaktionsgemisch auf Citratpuffer (pH 5), extrahiert mehrere Male mit Essigester, wäscht die organische Phase mit Sole, trocknet über Magnesiumsulfat und dampft im Vakuum ein. Nach Chromatographie des Rückstandes an Kieselgel mit Methylenchlorid erhält man 80 mg der Titelverbindung als Öl.

IR: 3600, 3400, 1718, 1600, 1590, 1125, 972/cm.

Beispiel 20

(13E)-(9R,11R,15R)-9-Chlor-11,15-dihydroxy-16-phenoxy-17,18,19,20-tetranor-13-prostensäure-methylsulfonamid

In Analogie zu Beispiel 19 erhält man aus der nach Beispiel 4 hergestellten Verbindung die Titelverbindung als Öl.
IR: 3605, 3410, 1720, 1600, 1588, 1125, 970/cm.

Beispiel 21

(5Z,13E)-(9R,11R,15R)-9-Chlor-11,15-dihydroxy-16-(m-chlorphenoxy)-17,18,19,20-tetranor-5,13-prostadiensäure-methylsulfonamid

In Analogie zu Beispiel 19 erhält man aus der nach Beispiel 6 hergestellten Verbindung die Titelverbindung als Öl.
IR: 3602, 3400, 1720, 1602, 1590, 1130, 970/cm.

Beispiel 22

(13E)-(9R,11R,15R)-9-Chlor-11,15-dihydroxy-16-(m-chlorphenoxy)-17,18,19,20-tetranor-13-prostensäure-methylsulfonamid

In Analogie zu Beispiel 19 erhält man aus der nach Beispiel 8 hergestellten Verbindung die Titelverbindung als Öl.
IR: 3602, 3400, 2960, 2870, 1720, 1601, 1590, 1125, 970/cm.

Beispiel 23

(5Z,13E)-(9R,11R,15R)-9-Chlor-11,15-dihydroxy-16,16-dimethyl-5,13-prostadiensäure-methylsulfonamid

Zu einer Lösung von 585 mg (5Z,13E)-(9R,11R,15R)-9-Chlor-16,16-dimethyl-11,15-bis-(tetrahydropyran-2-yloxy)-5,13-prostadiensäure (aus dem Methylester – siehe Beispiel 9 – durch Verseifung mit 1molarer Natronlauge in Methanol) in 25 ml Tetrahydrofuran fügt man 0,75 mg Methansulfonylisocyanat in 10 ml Tetrahydrofuran zu und rührt 6 Stunden bei 20°C. Anschließend neutralisiert man mit Essigsäure, engt im Vakuum ein, löst den Rückstand in 100 ml Methylenchlorid, schüttelt mit gesättigter Natriumhydrogencarbonatlösung und mit Wasser, trocknet über Magnesiumsulfat und dampft im Vakuum ein.

Zur Abspaltung der Schutzgruppen rührt man den Rückstand mit 10 ml einer Mischung aus Eisessig/Wasser/Tetrahydrofuran (65/35/10) 4 Stunden bei 40°C, dampft im Vakuum ein und absorbiert den Rückstand an 20 g Kieselgel. Durch Elution mit Hexan/Essigester (1 : 1) werden Verunreinigungen abgetrennt. Mit Essigester eluiert man dann 200 mg der Titelverbindung als Öl.
IR: 3600, 3420, 2955, 2868, 1718, 1120, 972/cm.

Beispiel 24

(5Z,13E)-(9R,11R,16RS)-9-Chlor-11,15α-dihydroxy-16-methyl-5,13-prostadiensäure-methylsulfonamid

In Analogie zu Beispiel 19 erhält man aus der nach Beispiel 12 hergestellten Verbindung die Titelverbindung als Öl.
IR: 3602, 3400, 1718, 1120, 972/cm.

Beispiel 25

(5Z,13E)-(9R,11R,15R)-9-Chlor-11,15-dihydroxy-16-phenoxy-17,18,19,20-tetranor-5,13-prostadiensäure-isopropylsulfonamid

200 mg (5Z,13E)-(9R,11R,15R)-9-Chlor-11,15-dihydroxy-16-phenoxy-17,18,19,20-tetranor-5,13-prostadiensäure (aus Beispiel 2) werden in 5 ml Dimethylformamid gelöst und bei 0°C mit 80 mg

Chlorameisensäureisobutylester und 60 mg Triäthylamin versetzt. Nach 30 Minuten werden 290 mg des Natriumsalzes von Isopropylsulfonamid (hergestellt aus Isopropylsulfonamid und Natriummethylat) und 2 ml Hexamethylphosphorsäuretriamid zugefügt und 3 Stunden bei 25° C gerührt. Zur Aufarbeitung gibt man auf 100 ml Citratpuffer (pH 5), extrahiert mehrmals mit Essigester, wäscht die organische Phase mit Sole, trocknet über Magnesiumsulfat und dampft im Vakuum ein. Nach Chromatographie des Rückstandes an Kieselgel mit Methylenchlorid erhält man 91 mg der Titelverbindung als Öl.

IR: 3600, 3410, 2960, 2870, 1722, 1601, 1588, 1120, 974/cm.

## Beispiel 26

(13E)-(9R,11R,15R)-9-Chlor-11,15-dihydroxy-16-phenoxy-17,18,19,20-tetranor-13-prostensäure-isopropylsulfonamid

In Analogie zu Beispiel 25 erhält man aus 200 mg (13E)-(9R,11R,15R)-9-Chlor-11,15-dihydroxy-16-phenoxy-17,18,19,20-tetranor-13-prostensäure (Herstellung siehe Beispiel 4) 85 mg der Titelverbindung als Öl.

IR: 3605, 3410, 2955, 2865, 1722, 1600, 1588, 1125, 974/cm.

## Beispiel 27

(5Z,13E)-(9R,11R,15R)-9-Chlor-11,15-dihydroxy-16-phenoxy-17,18,19,20-tetranor-5,13-prostadiensäure-acetylamid

Zu einer Lösung von 575 mg (5Z,13E)-(9R,11R,15R)-9-Chlor-11,15-bis-(tetrahydropyran-2-yloxy)-16-phenoxy-17,18,19,20-tetranor-5,13-prostadiensäure (hergestellt aus dem entsprechenden Methylester – siehe Beispiel 1 – durch Verseifung mit 1molarer Natronlauge in Methanol) in 15 ml Acetonitril gibt man bei 25° C 150 mg Triäthylamin, kühlt auf 0° C und tropft eine Lösung von 106 mg Acetylisocyanat in 10 ml Acetonitril zu. Anschließend rührt man 2 Stunden bei 25° C, engt im Vakuum ein, verdünnt mit 100 ml Wasser, stellt durch Zugabe von 1 N-Schwefelsäure auf pH 5 ein, extrahiert mehrmals mit Äther, wäscht die organische Phase mit Sole, trocknet über Magnesiumsulfat und dampft im Vakuum ein. Zur Abspaltung der Schutzgruppe rührt man den Rückstand mit 15 ml Eisessig/Wasser/Tetrahydrofuran (65/35/10) 5 Stunden bei 40° C und dampft dann im Vakuum zur Trockne. Der Rückstand wird an Kieselgel mit Methylenchlorid/1% Isopropylalkohol chromatographiert. Man erhält 220 mg der Titelverbindung als Öl.

IR: 3600, 3400, 2945, 2862, 1708, 1600, 1588, 976/cm.

## Beispiel 28

(13E)-(9R,11R,15R)-9-Chlor-11,15-dihydroxy-16-phenoxy-17,18,19,20-tetranor-13-prostensäure-acetylamid

In Analogie zu Beispiel 27 erhält man aus 450 mg (13E)-(9R,11R,15R)-9-Chlor-11,15-bis-(tetrahydropyran-2-yloxy)-16-phenoxy-17,18,19,20-tetranor-13-prostensäure (hergestellt aus dem entsprechenden Methylester – siehe Beispiel 3 – durch Verseifung mit 1molarer Natronlauge in Methanol) 200 mg der Titelverbindung als Öl.

IR: 3600, 3410, 2950, 2860, 1706, 1600, 1590, 976/cm.

## Beispiel 29

(13E)-(9R,11R,15R)-9-Chlor-11,15-dihydroxy-16-(m-chlorphenoxy)-17,18,19,20-tetranor-13-prostensäure-acetylamid

In Analogie zu Beispiel 27 erhält man aus 485 mg (13E)-(9R,11R,15R)-9-Chlor-11,15-bis-(tetrahydropyran-2-yloxy)-16-(m-chlorphenoxy)-17,18,19,20-tetranor-13-prostensäure (hergestellt aus dem entsprechenden Methylester – siehe Beispiel 7 – durch Verseifung mit 1molarer Natronlauge in Methanol) 225 mg der Titelverbindung als Öl.

IR: 3600, 3400, 2948, 2858, 1706, 1602, 1590, 976/cm.

## Beispiel 30

(5Z,13E)-(9R,11R,15R)-9-Chlor-11,15-dihydroxy-16-phenoxy-17,18,19,20-tetranor-5,13-prostadiensäure-amid

400 mg (5Z,13E)-(9R,11R,15R)-9-Chlor-11,15-dihydroxy-16-phenoxy-17,18,19,20-tetranor-5,13-prostadiensäure (Herstellung siehe Beispiel 2) werden in 10 ml Tetrahydrofuran gelöst und bei 0°C mit 140 mg Triäthylamin und 171 mg Chlorameisensäureisobutylester versetzt. Nach 1 Stunde wird bei 0°C 10 Minuten Ammoniakgas eingeleitet, dann 1 Stunde bei 25°C stehengelassen. Anschließend wird mit 50 ml Wasser verdünnt, dreimal mit je 50 ml Methylenchlorid extrahiert, die vereinigten Extrakte einmal mit 20 ml Sole geschüttelt, über Magnesiumsulfat getrocknet und im Vakuum eingedampft. Zur Reinigung wird der Rückstand an Kieselgel mit Chloroform/Essigester-Gemische chromatographiert. Man erhält 310 mg der Titelverbindung als wachsartige Masse.

IR: 3600, 3535, 3415, 2995, 2930, 2860, 1675, 1600, 1588, 972/cm.

## Beispiel 31

(13E)-(9R,11R,15R)-9-Chlor-11,15-dihydroxy-16-phenoxy-17,18,19,20-tetranor-13-prostensäure-amid

In Analogie zu Beispiel 30 erhält man aus der nach Beispiel 4 hergestellten Säure die Titelverbindung als Öl.

IR: 3600, 3535, 3410, 2996, 2930, 2860, 1670, 1601, 1588, 972/cm.

## Beispiel 32

(5Z,13E)-(9R,11R,15R)-9-Chlor-11,15-dihydroxy-16-(m-chlorphenoxy)-17,18,19,20-tetranor-5,13-prostadiensäure-amid

In Analogie zu Beispiel 30 erhält man aus der nach Beispiel 6 hergestellten Säure die Titelverbindung als Öl.

IR: 3600, 3450, 2998, 2930, 2862, 1670, 1600, 1585, 974/cm.

## Beispiel 33

(13E)-(9R,11R,15R)-9-Chlor-11,15-dihydroxy-16-(m-chlorphenoxy)-17,18,19,20-tetranor-13-prostensäure-amid

In Analogie zu Beispiel 30 erhält man aus der nach Beispiel 8 hergestellten Säure die Titelverbindung als Öl.

IR: 3600, 3420, 2998, 2935, 2860, 1672, 1600, 1588, 972/cm.

## Beispiel 34

(13E)-(9R,11R,15R)-9-Chlor-11,15-dihydroxy-16-phenoxy-17,18,19,20-tetranor-13-prostensäure-trishydroxymethylaminomethansalz

Zu einer Lösung von 410 mg (13E)-(9R,11R,15R)-9-Chlor-11,15-dihydroxy-16-phenoxy-17,18,19,20-tetranor-13-prostensäure (Herstellung siehe Beispiel 4) in 70 ml Acetonitril gibt man bei 65°C eine Lösung von 122 mg Tris-(hydroxymethyl)-aminomethan in 0,4 ml Wasser. Man läßt unter Rühren abkühlen, dekantiert nach 16 Stunden vom Lösungsmittel und trocknet den Rückstand im Vakuum. Man erhält 365 mg der Titelverbindung als weiße wachsartige Masse.

**Patentansprüche**

1. 9-Chlorprostaglandinderivate der allgemeinen Formel I

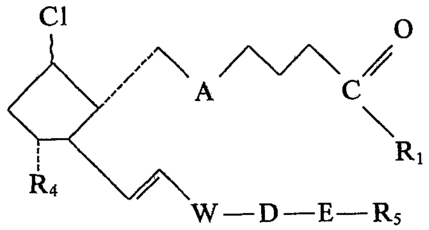

(I)

worin das 9-Chloratom $\alpha$- oder $\beta$-ständig sein kann,

$R_1$ den Rest $OR_2$ mit $R_2$ in der Bedeutung eines Wasserstoffatoms oder eines Alkyls mit 1–4 C-Atomen oder den Rest $NHR_3$ mit $R_3$ in der Bedeutung eines Wasserstoffatoms oder eines Carbon- oder Sulfonsäurerestes mit 1–10 C-Atomen,

A eine $-CH_2-CH_2$- oder cis-$CH=CH$-Gruppe,

W eine freie oder funktionell abgewandelte Hydroxymethylengruppe, wenn die Gruppierung $-DER_5$ nicht den Rest n-Pentyl darstellt, oder eine freie oder funktionell abgewandelte

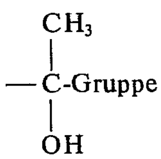

wobei die OH-Gruppen $\alpha$- oder $\beta$-ständig sein können,

D und E gemeinsam eine direkte Bindung oder

D eine geradkettige oder verzweigtkettige Alkylengruppe mit 1–5 C-Atomen, die gegebenenfalls durch Fluoratome substituiert ist,

und

E ein Sauerstoffatom oder eine direkte Bindung und

$R_4$ eine freie oder funktionell abgewandelte Hydroxygruppe,

$R_5$ eine gerad- oder verzweigtkettige, gesättigte oder ungesättigte Alkylengruppe mit 1–6 C-Atomen, die durch Halogen substituiert sein kann, oder eine gegebenenfalls durch 1–3 Halogenatome oder eine Trifluormethylgruppe substituierte Phenyl-Gruppe und falls $R_1$ die Bedeutung einer Hydroxylgruppe hat, deren Salze mit physiologisch verträglichen Basen bedeuten.

2. (5Z,13E)-(9R,11R,15R)-9-Chlor-11,15-dihydroxy-16-phenoxy-17,18,19,20-tetranor-5,13-prostadiensäure.

3. (13E)-(9R,11R,15R)-9-Chlor-11,15-dihydroxy-16-phenoxy-17,18,19,20-tetranor-13-prostansäure.

4. (5Z,13E)-(9R,11R,15R)-9-Chlor-11,15-dihydroxy-16,16-dimethyl-5,13-prostadiensäure-methylester.

5. (5Z,13E)-(9R,11R,15R)-9-Chlor-11,15-dihydroxy-16,16-dimethyl-5,13-prostadiensäure.

6. (5Z,13E)-(9R,11R,15R)-9-Chlor-11,15-dihydroxy-16,16-dimethyl-5,13-prostadiensäure-methylsulfonamid.

7. (13E)-(9R,11R,15R)-9-Chlor-11,15-dihydroxy-16-phenoxy-17,18,19,20-tetranor-13-prostensäure-trishydroxymethylaminomethansalz.

8. Verfahren zur Herstellung der 9-Chlorprostaglandinderivate der allgemeinen Formel I, dadurch gekennzeichnet, daß man in an sich bekannter Weise eine Verbindung der allgemeinen Formel II

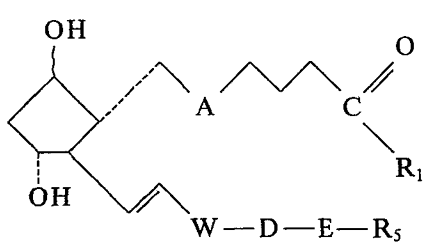

(II)

worin die OH-Gruppen $\alpha$- oder $\beta$-ständig sein können, $R_1$, A, D, E und $R_5$ die obengenannte Bedeutung haben und freie OH-Gruppen in $R_4$ und W geschützt sind,

a) über einen intermediären Sulfonsäureester mit einem Chlorid der allgemeinen Formel III $R_6Cl$, worin $R_6$ die Bedeutung Lithium, Natrium, Kalium oder Tetraalkylammonium mit Alkyl als gesättigtem $C_1-C_6$-Rest hat, umsetzt oder

b) mit dem Reagenz Tetrachlorkohlenstoff/Triphenylphosphin chloriert und gegebenenfalls anschließend in den nach a) oder b) erhaltenen Reaktionsprodukten in beliebiger Reihenfolge geschützte Hydroxygruppen freisetzt und/oder Doppelbindungen hydriert und/oder eine veresterte Carboxylgruppe verseift und/oder eine freie Carboxylgruppe verestert und/oder eine $COR_1$-Gruppe mit $R_1=OH$ in eine $CONHR_3$-Gruppe in der oben angegebenen Bedeutung umwandelt.

9. Arzneimittel, bestehend aus einer oder mehreren Verbindungen gemäß Anspruch 1 und üblichen Hilfs- und Trägerstoffen.

**Claims**

1. 9-chloroprostaglandin derivates of the general formula I

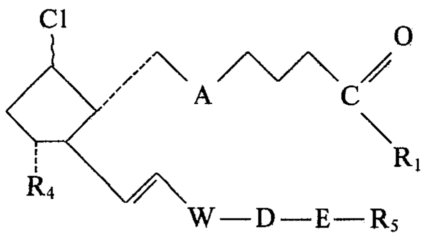

(I)

in which
the 9-chlorine atom can be in the $\alpha$-configuration or the $\beta$-configuration,

$R_1$ represents the radical $OR_2$, in which $R_2$ represents a hydrogen atom or an alkyl having from 1 to 4 carbon atoms; or the radical $NHR_3$, in which $R_3$ represents a hydrogen atom or a carboxylic acid radical or a sulphonic acid radical having from 1 to 10 carbon atoms;

A represents a $-CH_2-CH_2$-group or a cis-$CH=CH$-group,

W represents a free or functionally modified hydroxymethylene group, if the grouping $-DER_5$ does not represent the n-pentyl radical, or a free or functionally modified

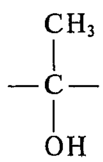
-group

in which the OH groups can be in the $\alpha$-configuration or the $\beta$-configuration,

D and E together represent a direct bond, or

D represents a straight-chain or branched-chain alkylene group having from 1 to 5 carbon atoms which is optionally substituted by fluorine atoms, and

E represents an oxygen atom or a direct bond and

$R_4$ represents a free or functionally modified hydroxy group,

$R_5$ represents a straight-chain or branched-chain, saturated or unsaturated alkyl group having from 1 to 6 carbon atoms, which can be substituted by halogen, or a phenyl group which is optionally substituted by from 1 to 3 halogen atoms or by a trifluoromethyl group and,
if $R_1$ represents a hydroxy group, the salts thereof with physiologically tolerable bases.

2. (5Z,13E)-(9R,11R,15R)-9-chloro-11,15-dihydroxy-16-phenoxy-17,18,19,20-tetranor-5,13-prostadienoic acid.

3. (13E)-(9R,11R,15R)-9-chloro-11,15-dihydroxy-16-phenoxy-17,18,19,20-tetranor-13-prostenoic acid.

4. (5Z,13E)-(9R,11R,15R)-9-chloro-11,15-dihydroxy-16,16-dimethyl-5,13-prostadienoic acid methyl ester.

5. (5Z,13E)-(9R,11R,15R)-9-chloro-11,15-dihydroxy-16,16-dimethyl-5,13-prostadienoic acid.

6. (5Z,13E)-(9R,11R,15R)-9-chloro-11,15-dihydroxy-16,16-dimethyl-5,13-prostadienoic acid methylsulphonamide.

7. (13E)-(9R,11R,15R)-9-chloro-11,15-dihydroxy-16-phenoxy-17,18,19,20-tetranor-13-prostenoic acid trishydroxymethylaminomethane salt.

8. Process for the manufacture of the 9-chloro-prostaglandin derivatives of the general formula I,

characterised in that, in a manner known per se, a compound of the general formula II

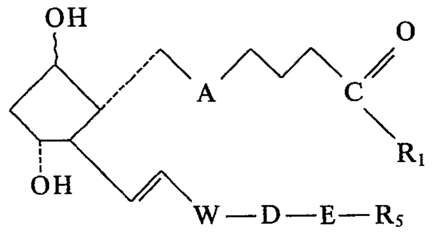

(II)

in which the OH groups can be in the α-configuration or the β-configuration, $R_1$, A, D, E and $R_5$ have the above-mentioned meanings and free OH groupe in $R_4$ and W are protected, is

a) reacted via an intermediate sulphonic acid ester with a chloride of the general formula III $R_6Cl$, in which $R_6$ represents lithium, sodium, potassium or tetraalkylammonium with alkyl being a saturated $C_1-C_6$ radical, or

b) chlorinated with the reagent carbon tetrachloride/triphenylphosphine and then, optionally, in the reaction products obtained in accordance with a) or b), in any desired order, protected hydroxy groups are freed and/or double bonds are hydrogenated and/or an esterified carboxy group is hydrolysed and/or a free carboxy group is esterified and/or a $COR_1$ group in which $R_1=OH$ is converted into a $CONHR_3$ group having the above-mentioned meaning.

9. Medicament comprising one or more compounds according to claim 1 and customary auxiliaries and carriers.

**Revendications**

1. Chloro-9 prostaglandines répondant à la formule générale I

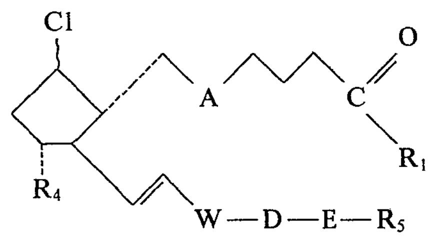

(I)

dans laquelle
l'atome de chlore en 9 a la configuration α ou la configuration β,

$R_1$ représente un radical $OR_2$ dans lequel $R_2$ désigne un atome d'hydrogène ou un alkyle en $C_1-C_4$, ou représente un radical $NHR_3$ dans lequel $R_3$ désigne un atome d'hydrogène ou un radical d'acide carboxylique ou d'acide sulfonique en $C_1-C_{10}$,

A représente un radical $-CH_2-CH_2-$ ou un radical $-CH=CH$-cis,

W représente un radical hydroxyméthylène libre ou sous la forme d'un dérivé fonctionnel dans le cas où le groupement $-D-E-R_5$ n'est pas un radical n-pentyle, ou représente un radical

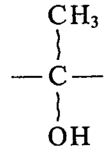

libre ou sous la forme d'un dérivé fonctionnel, les radicaux OH ayant la configuration α ou la configuration β,

D et E forment ensemble une liaison directe ou

D représente un radical alkylène, linéaire ou ramifié, qui contient de 1 à 5 atomes de carbone et qui porte éventuellement des atomes de fluor, et

E représente un atome d'oxygène ou une liaison directe,

$R_4$ représente un groupe hydroxy libre ou sous la forme d'un dérivé fonctionnel, et

$R_5$ représente un radical alkyle en $C_1-C_6$, linéaire ou ramifié, saturé ou insaturé, qui peut porter un halogène, ou représente un radical phényle éventuellement porteur d'1 à 3 atomes d'halogène ou

d'un radical trifluorométhyle,
et, lorsque $R_1$ est un radical hydroxy, les sels que peuvent alors former ces composés avec des bases acceptables du point de vue physiologique.

2. Acide chloro-9 dihydroxy-11,15 phénoxy-16 tétranor-17,18,19,20 prostadiène-5,13 oïque (5Z,13E)-(9R,11R,15R).
3. Acide chloro-9 dihydroxy-11,15 phénoxy-16 tétranor-17,18,19,20 prostène-13 oïque (13E)-(9R,11R,15R).
4. Ester méthylique de l'acide chloro-9 dihydroxy-11,15 diméthyl-16,16 prostadiène-5,13 oïque (5Z,13E)-(9R,11R,15R).
5. Acide chloro-9 dihydroxy-11,15 diméthyl-16,16 prostadiène-5,13 oïque (5Z,13E)-(9R,11R,15R).
6. Méthylsulfonylamide de l'acide chloro-9 dihydroxy-11,15 diméthyl-16,16 prostadiène-5,13 oïque (5Z,13E)-(9R,11R,15R).
7. Sel de l'acide chloro-9 dihydroxy-11,15 phénoxy-16 tétranor-17,18,19,20 prostène-13 oïque (13E)-(9R,11R,15R) avec le tris-(hydroxyméthyl-aminométhane.
8. Procédé de préparation des chloro-9 prostaglandines de formule générale I, procédé caractérisé en ce que, en opérant de manière connue:

a) on fait réagir un composé répondant à la formule générale II

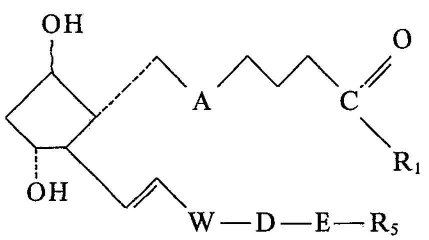

(II)

dans laquelle les radicaux OH ont la configuration $\alpha$ ou $\beta$, $R_1$, A, D, E et $R_5$ ont les significations précédemment données et les radicaux OH que peuvent renfermer $R_4$ et W sont protégés, ce composé (II) ayant au préalable été estérifié intermédiairement par un acide sulfonique, avec un chlorure de formule générale $R_6Cl$ (III) dans lequel $R_6$ représente le lithium, le sodium, le potassium ou un radical tétra-alkylammonium dont les alkyles sont saturés et contiennent chacun de 1 à 6 atomes de carbone, ou

b) on chlore un composé de formule générale II au moyen du réactif constitué de tétrachlorure de carbone et de triphénylphosphine,
et ensuite, sur les produits réactionnels obtenus selon (a) ou selon (b), on effectue éventuellement, dans l'ordre que l'on veut, les réactions suivantes: on libère des groupes hydroxy protégés et/ou on hydrogène des doubles liaisons et/ou on saponifie un radical carboxy estérifié et/ou on estérifie un radical carboxy libre et/ou on transforme un radical $-COR_1$ dans lequel $R_1$ désigne un $-OH$ en un radical $-CONHR_3$ tel que défini ci-dessus.

9. Médicament constitué d'un ou plusieurs composés selon la revendication 1 et d'adjuvants et excipients usuels.